# EUROPEAN PATENT SPECIFICATION

(11) **EP 2 717 880 B1**
(45) Date of publication and mention of the grant of the patent: **14.12.2016**
(21) Application number: 12724984.5
(22) Date of filing: 30.05.2012
(51) Int. Cl.: A61K 31/57, A61P 5/36, A61K 45/06, A61K 9/00, A61K 31/567

(54) **SELECTIVE CNS DELIVERY OF MIFEPRISTONE (RU486) TO MODULATE THE TIMING OF THE SPONTANEOUS LH SURGE DURING FOLLICULAR STIMULATION CYCLES**
SELEKTIVE ZNS-FREISETZUNG VON MIFEPRISTON (RU486) ZUR MODULIERUNG DES ZEITPUNKTES DES SPONTANEN LH-ANSTEIGS WÄHREND FOLLIKULARER STIMULATIONSZYKLEN
ADMINISTRATION AU SYSTÈME NERVEUX CENTRAL (SNC) SÉLECTIVE DE MIFÉPRISTONE (RU486) POUR MODULER LA CHRONORÉGULATION DE L'AUGMENTATION SPONTANÉE DE LH AU COURS DES CYCLES DE STIMULATION FOLLICULAIRE

(30) Priority: 13.06.2011 IT VA20110016
(43) Date of publication of application: 16.04.2014
(73) Proprietor: Parthenogen SAGL, 6900 Lugano (CH)
(72) Inventor: DATTILO, Maurizio, CH-6926 Montagnola (CH)
(74) Representative: Pistolesi, Roberto
(86) International application number: PCT/EP2012/060122
(87) International publication number: WO 2012/171793

(56) References cited:
- WO-A1-03/055524
- US-A1- 2007 213 306
- US-A1- 2010 261 693

## Description

The present invention relates to a new pharmaceutical product containing an antiprogestin molecule, such as mifepristone (RU486), administered by the nasal route and to be used to modulate the timing of the spontaneous LH surge as part of follicular stimulation cycles intended for Assisted Reproductive Technology (ART). In particular, it details the route of administration and the dosages that ensure effective delay of the LH surge without incurring in the antifolliculogenetic effects of the antiprogestinic. It also details the overall treatment method as resulting from the use of the new drug product.

### Field of application

This invention relates to the area of Assisted Reproductive Technology (ART) and in particular to the modalities of stimulation of the women ovarian follicles. In particular it describes a new pharmaceutical product containing an antiprogestin such as mifepristone (RU486) delivered by the nasal route to ensure CNS preferential delivery. The new drug product is to be used to delay the occurrence of the spontaneous gonadotrophin surge during follicular stimulation treatments intended for assisted reproduction purposes. In a complementary aspect the present invention details the new overall treatment strategy resulting from the implementation of the new product.

### Prior art

Ovarian follicles are stimulated for reproductive purposes to sustain the growth and maturation of either a single follicle or multiple follicles. This is a critical step in vitro fertilization therapy that, in spite of decades of experience and developments, still remains critical and open to improvements¹.

In the single ovulation induction protocols the development of a single dominant follicle is obtained by administrating variable, although small, doses of drugs containing the Follicular Stimulating Hormone (FSH) or any of its agonists. Once the maturation of a single follicle is reached the pregnancy may be obtained by either timed intercourses or intrauterine insemination (IUI) with the partner's sperms.

In other instances, i.e. for Assisted Reproductive Technology (ART) purposes, the treatment goal is the development of multiple follicles and this requires a so-called Controlled Ovarian Hyperstimulation (COH). In this case supra-physiological doses of FSH or any FSH agonists are administered so that the sensitivity threshold of several follicles is reached. At the end of the stimulation, i.e. when the targeted number of follicles has been developed, the oocytes contained in these follicles are collected by needle aspiration, usually through the vaginal route. These oocytes will be used for In Vitro Fertilisation (IVF) or for Intra Cytoplasmic Sperm Injection (ICSI) to obtain viable embryos that will be transferred into the uterus of the mother in the hope that they will implant to give a pregnancy.

The main problem in performing these treatments is that, when follicles maturation approaches and the estradiol circulating levels become high, the pituitary gland will release a massive surge of Luteinising Hormone (LH) that triggers the final maturation and rupture of the follicles with consequent release of the contained oocytes into the fallopian tube. If the spontaneous LH surge occurs during a COH cycle, the oocytes are lost to the planned pick-up and the cycle must be cancelled.

This problem is currently managed by inhibiting the activity of the pituitary gland by administering either an agonist or an antagonist of the hypothalamic Gonadotrophin Releasing Hormone (GnRH)².

The GnRH-agonists induce an initial flare-up of the secretion of FSH and LH, thereafter a down-regulation of the GnRH-receptors occurs and the pituitary activity remains blocked at variable degrees according to the treatment schedule used. Inasmuch, the actual pituitary suppression will be reached only after some days of treatment and will last far long after the completion of the intended COH cycle leaving the patient in a state of chemical castration.

In contrast, the GnRH-antagonists act by competitively inhibiting the GnRH-receptors. Thus, their activity begins immediately after the start of dosing and will terminate shortly after withdrawal. In this respect the GnRH-antagonists carry a clear-cut advantage over the agonists, nevertheless the clinical results may be less brilliant due to excess of suppression of the LH axis (defect of oocyte maturation) and/or to negative effects on the endometrial mucosa (defect in implantation).

However, even though necessary for a successful cycle, the drawback of every pituitary suppression within COH regimens is the loss of the pituitary regulation of the complex endocrine background. Indeed, the pituitary responds to a series of peripheral feedbacks including, among the others, the circulating steroid hormones, the inhibin and activin system. In response to these feedbacks the gland releases FSH and LH according to defined quantitative and qualitative patterns. In particular, the LH secretion is pulsatile and the frequency, amplitude and LH isoforms composition of the pulses is regulated by the above feedbacks and will never be appropriately substituted by bolus injections of LH agonists. As a matter of fact, once the pituitary activity is suppressed, the entire system depends on the administration of exogenous gonadotrophins that can not completely substitute the natural secretions.

Another issue related to the suppression of the pituitary activity is that, once the COH treatment has duly developed the targeted oocytes, the natural LH ovulatory peak will not occur and the final maturation of the follicles will have to be artificially induced. The most widely used and validated strategy to induce the final maturation is the injection of a bolus of five to ten thousands IU of the human Chorionic Gonadotrophin (hCG). This placental hormone has a structure similar to LH and binds the same receptors, which is why it is used to substitute the natural LH. The problem is that LH is a short half-life hormone, i.e. about 2 hours, consistently with its fine tuning role, whereas hCG has a half-life in the range of 72 hours. Therefore, the hCG bolus, besides efficiently inducing the follicles maturation and rupture, also induces a LH-like activity that is sustained over time and that may not allow a perfectly timed shift from the follicular to the luteal phase of the cycle.

In summary, pituitary suppression, although currently necessary for an effective COH cycle, carries a series of drawbacks that remain a bill paid to the quality of the stimulation and to the final efficacy of the ART practice. Accordingly, alternative strategies for the prevention of the spontaneous LH surge would be clinically useful³.

In this respect, a possible alternative to the GnRH antagonism is the modulation of the mechanisms that regulate the release of GnRH. Indeed, the LH surge is directly induced by a surge of GnRH. As recently reviewed by Micevyich and Sinchak (2008)⁴ such mechanisms include an action of the estradiol of ovarian origin in the brain. Briefly, when ovarian estradiol rises at high concentrations sustained over time, i.e. during the late follicular phase, it induces progesterone receptors in the hypothalamus and also binds the estrogen receptor alpha of the astrocytes to induce the synthesis of neuroprogesterone that will diffuse from the astrocytes to local neurons. It is also possible that progesterone of follicular origin, that appears at time of follicle maturation, enters the Central Nervous System (CNS) to further sustain the activation of the system. As a matter of fact, in presence of hypothalamic progesterone receptors, induced by sustained estradiolemia, and of free progesterone in the CNS, likely both estrogen-induced neuroprogesterone and follicular progesterone, the hypothalamus changes its pattern of release of GnRH from pulsatile to massive and generate the as well massive release of LH from the pituitary that constitutes the ovulatory surge.

The above described regulatory mechanisms allow the hypothesis that the GnRH surge as well as the LH surge could be prevented with the administration of a progesterone antagonist.

Mifepristone (17β -hydroxyl-11β -(4-dimethyl-aminophenyl-1)-17α -(prop-1-ynyl)-estra-4,9-diene-3-one), also known as RU486, was the first clinically available progesterone receptor antagonist⁵. Thereafter, many other similar compounds have been synthesized. It is clinically indicated, together with prostaglandins, for the induction of medical abortion, which usually requires the daily oral administration of 200 mg for up to 3 consecutive days.

Already in 1989 Permezel et al.⁶ investigated the potential of progesterone to initiate the gonadotrophin surge and the ability of mifepristone to antagonise such effect. They found that 10 mg progesterone i.m. given in mid- or late-follicular phase generated an acute increase of plasma FSH and LH concentration and that this increase was in direct correlation with the baseline estradiol level. In contrast, the women receiving 10 or 100 mg of mifepristone per os had a reduction of FSH and LH plasma concentration, the effect being more evident when the drug was given in the late follicular phase.

A few years later Batista et al. (1992)⁷ administered mifepristone, 1 mg per os, starting when the dominant follicle had reached a diameter of 14-16 mm to 6 normally cycling women for 5 days and found that the circulating levels of FSH and LH remained flat while the follicle development pattern was the same of the placebo group. The LH surge occurred within 1 to 3 days in the placebo group but was delayed in all treated women and occurred only 2 or 3 days after discontinuing mifepristone. They repeated the same mifepristone administration to another group of 6 normally cycling women and then, when the leading follicle had reached a diameter of 20 mm (which occurred 3 to 5 days later), progesterone i.m. 2.5 mg bid on the first day and 5 mg bid on the second day was given. A typical gonadotrophin surge occurred within one day from the first progesterone injection in 5 out of 6 women and one day later in the sixth woman.

However, when Croxatto et al. (1995)⁸ administered 5 mg of mifepristone per day starting at mid follicular phase, they observed that even a single dose had caused an arrest of follicular growth, which they interpreted as an antifolliculotropic effect related to an interference with the FSH-granulosa cells axis. It can be today commented that their dose of mifepristone, although effective in inhibiting the gonadotrophin surge, was too high and too early to avoid interference with folliculogenesis.

Several of the above concepts have been considered by Coelingh Bennink & Bunschoten⁹ in their patent EP1455831 ("Method of controlled ovarian hyperstimulation and pharmaceutical kit for use in such method"), even though they missed to understand and factor for several main issues.

Briefly, Coelingh Bennink & Bunschoten claimed for a pharmaceutical composition containing an antiprogestinic to be used within a treatment method including: i) a FSH preparation to be administered from the beginning of the cycle according to the usual schedule; ii) an antiprogestin to be associated to FSH beginning when the leading follicle is larger than 14 mm (preferably 12 mm, more preferably 10 mm) and ending 1 day prior the administration of a ML (meiosis resumption and luteinising) substance (Claim 6), or at least from day 6 of stimulation up to 4 days before the administration of the ML substance (claim 7); iii) A ML substance to be administered only once, at time of FSH discontinuation, to induce the final maturation of the follicles, and; iv) a progestin to be administered starting from the day of the ML substance bolus to counteract the negative effect of the anti-progestin on the endometrial mucosa. In practise, assumed that points i), iii) and iv) are the standard practise in these treatments, the innovation was point ii), i.e. the administration of an antiprogestin. Such administration, as proposed and claimed by Coelingh Bennink & Bunschoten was based on questionable concepts and unlikely to be applicable to the clinical practice.

According to Coelingh Bennink & Bunschoten the antiprogestin treatment is to be started not later then day 6 of stimulation and however when the follicles are still small (less than 14 mm) to be sure that it will be protecting the endometrial mucosa from the negative effects of the follicular progesterone, that appears at time of maturation. Thus, they assume that the negative predictivity associated to the emergence of follicular progesterone is related to its endometrial effects, which is untrue. The progesterone plasma concentration at the end of a COH cycle is reported by Andersen AN et al. (2006)¹⁰ to be between 2 and 5 nmol/L, which is at least one order of magnitude lower than the progesterone concentration inducing secretory changes in the endometrium in a standard luteal phase. Andersen et al. (2006)¹⁰ divided their patients according to a post-hoc cut-off value of 4 nmol/L of circulating progesterone to find that the subgroup with end-of-stimulation progesterone higher than 4 nmol/L had only slight, possibly non clinically significant, changes in the endometrium whereas there was a strong trend for a lower global efficacy, likely due to some kind of oocyte defect. Indeed, Coelingh Bennink & Bunschoten failed to understand that the appearance of progesterone secretion is just the signal that the follicle is ready for final maturation, being itself the signal for the release of the LH surge and being this signal effective at very low progesterone concentration, as it is acting as a neurotransmitter. Accordingly, the negative outcome related to high end-of-cycle progesterone is due to the fact that the leading follicles, that had already started to release progesterone, are somehow overmature at time of oocyte pick-up. In conclusion, the administration of the antiprogestin early in the cycle will not produce any endometrial protection and will just expose the cycle to the antifolliculogenetic activity of the antiprogestin.

Thus, in contrast with EP1455831, the antiprogestin administration should start as late as possible, i.e. only when there is high risk that the hyperstimulated follicles will start releasing progesterone, which is when they reach the size of 20 mm, usually by day 8 of stimulation or later.

According to Coelingh Bennink & Bunschoten the treatment cycle will have to be concluded with the administration of a "ML substance" that can be either hCG or recombinant LH or a GnRH agonist. Again, they fail to understand a main added value of the surge inhibition by the antiprogestin that is the possibility to have a pituitary-generated gonadotrophin surge. Indeed, the pituitary is able to read the endocrine background and to release the right amount and the appropriate isoforms of LH and, in case, of FSH giving a gonadotrophin surge that would never be paralleled by the currently available agonists. As shown by Batista et al. (1992)⁷ the pituitary surge was spontaneously released without any triggering within 2 to 3 days after mifepristone withdrawal in all treated patients. This interval was shortened to 1 day when a triggering, although very small, dose of progesterone was given. Thus, the antiprogestin-managed COH cycle should definitely be not triggered or progesterone triggered. With reference to the latter intervention, it is also expected that a strategy of progesterone administration more selective for the CNS would result in a far large therapeutic window.

According to Coelingh Bennink & Bunschoten the antiprogestin is to be administered at a daily oral dose of 0.1-42 mg, preferably of 0.2-22 mg mifepristone, more preferably of 0.5-12 mg mifepristone (Claim 2). Such dose range is tremendously large and includes at one end dosages that are very likely to be ineffective by the oral rotute in hyperstimulated patients or, at the other end, proven to be antifolliculogenetic. Rather, the actual dosage that is at the same time effective in preventive the LH surge and safe for the follicles is certainly included within the range 1 to 5 mg daily by the oral route, which is indeed too narrow. However, such dose range might be different if alternative routes of administration are considered.

According to Coelingh Bennink & Bunschoten the antiprogestin, namely mifepristone, is to be given by the oral route, which is not appropriate. Indeed, they fail to consider that mifepristone is intended to antagonise progesterone receptors in the hypothalamus and that therefore its concentration in the CNS is the actual reference. The main issue for the delivery of substances to the CNS is the Blood-Brain Barrier (BBB)¹¹ that blocks large molecules and allow the passage of less then 2% of the small molecules. Thus, even assuming mifepristone as a small molecule, for each mg given by the oral route only 2% is supposed to enter the CNS to block the progesterone receptors, the rest is just follicular antagonism.

Therefore, the so far narrow therapeutic window, i.e. 1 to 5 mg per os, may be enlarged toward an actual clinical suitability by implementing a CNS selective delivery. This is possible by recurring to the nasal administration that has the potential to deliver part of the administered dose directly to the CNS. There are several mechanisms underlying the direct nose to brain drug delivery¹²⁻¹⁶. First, there is an intracellular transport with the olfactory neurones facing the epithelium capturing molecules by endocytosis and delivering them to the olfactory bulb via axonal transport. Second, there are intercellular pathways, through the gaps between the olfactory neurons or along the trigeminal nerve. Once in the olfactory bulb or in the trigeminal area, substances diffuse to the rest of the brain under a concentration gradient. Finally, substances may be drained by the lymphatic vessels into the CNS.

The effect size of a nose to brain administration compared to the standard systemic route may be huge. In a typical example, NAD⁺ that has the potential to prevent oxidative stress-induced cell death, was ineffective when given by injection following rat brain ischemia. Rather, it reduced by 90% the infarct formation when given by the nasal route¹⁷⁻¹⁸.

A main feature of molecules suitable for nasal absorption is lipid solubility, which is typical of steroid molecules. Accordingly, several of those molecules have been already successfully administered by the nasal route, including estrogens¹⁹ and progestins²⁰.

Mifepristone has a steroid-like structure and good lipid solubility and is as well suitable for nose to brain administration. Inasmuch, mifepristone given by this route may increase the rate of the drug bioavailable at the hypothalamic compartment while proportionally decreasing the rate available to the follicles and to the endometrium. The effective therapeutic doses could remain roughly the same but with the surge delaying efficacy occurring with less of antifolliculogenetic effects making the treatment actually suitable for clinical use.

In summary, it is clear from the state of the art that the prevention of the spontaneous LH ovulatory surge is necessary for an effective COH treatment within ART programs. It is also clear that the GnRH agonism/antagonism, although effective in preventing the surge, generates perturbations of the endocrine milieu that can not be completely reverted by the available treatments. On the other side there are literature evidences that the same LH surge could be prevented by the administration of low doses of a progesterone antagonist, where the available data are mostly based on the use of mifepristone. However, it is also clear that the main problem to overcome for an actual clinical use is the very narrow therapeutic window. Indeed, the systemic dose of mifepristone necessary to prevent the LH surge in all patients under COH treatment, likely higher than 1 mg by oral route⁷, is very close to the dose generating anti-folliculogenetic effects, which where already evident at 5 mg per day orally⁸. In this respect, the patent EP1455831 from Coelingh Bennink & Bunschoten did not address the question because the dose, route of administration and the overall clinical protocol they outlined does not appear to be appropriate for the scope. This is confirmed by the clinical study of Escudero EL et al. (2005)²¹, which was sponsored by the holders of patent EP1455831, that treated 10 patients undergoing a COH cycle with mifepristone, 40 mg oral route, since the early days of the FSH administration. All of them received 50 mg progesterome i.m. at the end of stimulation and 5 of them also received an injection of hCG for triggering. All mifepristone patients did not have a gonadotrophin surge. However, as expected from the too large dose and the too early administration of mifepristone, patients under mifepristone + progesterone did not give any mature oocyte whereas patients administered mifepristone + progesterone + hCG gave an average of 11.6 oocytes collected, almost half of the 19.6 oocytes collected in the control group (standard GnRH antagonist protocol). On top of this, mifepristone patients showed a negative endometrial evolution. This clearly indicated that their endometrial protection rationale was wrong and that they had reached an antifolliculogenetic exposure to mifepristone that had been only partially compensated by the hCG.

US 2010/261693²² discloses a method for treating the Cushing's syndrome by administering a glucocorticoid receptor antagonist, such as mifepristone, at least twice a day. Buccal, intradermal and nasal administration are listed among the possible ways of administration of the glucocorticoid receptor antagonist.

US 2007/213306²³ discloses a method of treating uterine leiomyomata, premenstrual syndrome, premenstrual dysphoric disorder, dysfunctional uterine bleeding, polycystic ovarian syndrome, adenomyomas, polymenorrhea, dysmenorrhea, severe dysmenorrhea, menorrhagia, breakthrough bleeding, intermittent bleeding, endometriosis, ovarian cysts, irregular withdrawal bleeding, hirsutism, iron deficiency anemia, acne, benign breast disease, catamenial symptoms, pelvic inflammatory disease, loss of bone density, endometriosis, breast cancer, ovarian cancer, uterine cancer, or prostate cancer by administering an anti-progestational agent, such as mifepristone. The anti-progestational agent may be administered by oral administration, sublingual administration, buccal administration, nasal administration, transdermal administration, vaginal administration, rectal administration, intravenous administration, subcutaneous administration, intraperitoneal administration, parenteral administration, intramuscular administration, depot administration, topical administration, intrauterine administration, inhalation administration, implant administration or ocular administration.

In conclusion, even if mifepristone has the full potential to be useful as a gonadotropin surge inhibitor, the current oral dosing does not appear to be clinically suitable. Moreover, nobody did ever link the modern understanding of the neuroendocrine mechanisms underlying the progesterone regulation of the ovulatory midcycle gonadotrophin peak with the clinical needs for an effective COH cycle. It is today possible to address such needs by changing the administration strategy of the antiprogestinic. In this respect, the nose to brain delivery appears as an entirely new and unexplored approach.

### Detailed description of the invention

Briefly stated, in one aspect, the present invention describes a new pharmaceutical product containing a progesterone antagonist to be administered by the nasal route.

In a preferred embodiment of the present invention the progesterone antagonist is intended to prevent the spontaneous mid cycle surge in COH treatments as part of ART treatments.

In another preferred embodiment of the present invention the progesterone antagonist contained in the new pharmaceutical product is mifepristone (RU486).

Mifepristone is a competitive inhibitor of the progesterone receptor (PR), which is the basic mechanism of action underlying the already approved indication for the induction of the medical aborption.

Among other functions, PRs are involved in a cyclic complex signalling between the ovary and the hypothalamus. The rising concentration of ovarian estradiol in the late follicular phase induces the exposure of PRs in the hypothalamus. Once progesterone comes available for binding, which occurs at the end of the follicular phase, the hypothalamus changes the pattern of release of its GnRH from pulsatile to massive. The surge of GnRH causes in turn a surge of gonadotrophins, especially of LH, from the pituitary. The above described event is known as ovulatory surge because under the stimulation of a huge amount of LH the oocyte of the leading ovarian follicle will resume the meiotic division and complete the maturation while the granulosa cells constituting the oocyte cumulus will loosen their junctions to finally liberate the mature oocyte into the tube.

Patients suffering from reproductive problems may need to undergo a Controlled Ovarian Hyperstimulation (COH) cycle as par of an ART program. In these cycles an early spontaneous gonadotrophin surge may cause an early release and loss of the oocytes, therefore it is necessary to keep the release of the surge under control.

Mifepristone, due to its PR competitive inhibition, has the potential to reversibly block the hypothalamic PR and thus to delay the occurrence of the gonadotrophin surge as long as needed for the success of the COH cycle. Such use has been already proven in published clinical studies. However, the PR antagonism of mifepristone is also responsible for antifolliculogenetic effects and in clinical studies the dose causing perturbation of the folliculogenesis was very close if not overlapping the dose effective in the prevention of the gonadotropin surge. This problem makes the therapeutic window of mifepristone in this indication too narrow for a realistic clinical suitability.

It has been now discovered that the rate of mifepristone bioavailable at the hypothalamic site can be increased at the expense of the rate reaching the circulation by administering mifepristone by the nasal route, which addresses the above outlined problem and constitutes the basic novelty of the present invention.

The nasal route has the potential to deliver molecules directly to the brain, i.e. by nose to brain route, by means of both intracellular and intercellular transport pathways and has already resulted in successful selective CNS delivery of drugs. Mifepristone, thanks to its high lipid solubility, is particularly suitable for nose to brain delivery, as already demonstrated for similarly lipid soluble steroids, i.e. estrogen and progesterone.

The present invention is therefore represented by a pharmaceutical product containing an antiprogestinic molecule for use in the temporary suppression of the spontaneous midcycle gonadotrophin surge in women undergoing follicular stimulation for Assisted Reproduction (ART) purposes by delivery of the antiprogestinic molecule to the CNS, characterized in that it is to be administered by nasal route starting from the day the follicles reach the size of 16 mm in diameter.

In a preferred embodiment of the present invention mifepristone is formulated for nasal administration as a liquid solution in organic solvents including, but not limited to, ethanol and dimethyl sulfoxide (DMSO), both capable to dissolve more than 20mg/ml of mifepristone. In alternative, mifepristone is formulated as a solution in organic oil.

Even though preferentially delivered to the CNS, mifepristone may keep some antifolliculogenetic potential, thus it is necessary to limit its administration only to the days of the COH cycle that are at real risk of early occurrence of the gonadotrophin surge. Such risk became real when the first leading follicle within the stimulated cohort reaches maturity because it will start releasing small amounts of progesterone that may be however enough to trigger a system already strongly primed by high estrogens. Such level of maturity is certainly not reached until the follicle has a diameter of 16 mm and it is from this time that mifepristone nasal administration should start. However, in the sake of full follicular safety and if precise ultrasonografic measurement is available, it may be advisable to slightly delay the start of mifepristone dosing up to the time follicles reach sizes of 20 mm.

In a preferred embodiment of the present invention mifepristone nasal delivery starts when the leading follicle reach the diameter of 18 mm, preferably 20 mm. In any case, mifepristone dosing should not start later than day 8 of FSH administration within COH cycles.

Once administered by the nasal, i.e. nose to brain, route, only a small part of the total dose, but likely more than the 2% supposed to be delivered to the CNS by an oral administration, will be bioavailable at the hypothalamic compartment by a direct nose to brain pathway. However, the IC₅₀ for mifepristone-induced competitive binding to PR wild type receptor is as low as 0.1 nM²⁴. Thus, assumed the small volume of distribution of the CNS, roughly one liter, the actual absorption and transport into the CNS of just 50 nanograms of mifepristone, equal to 0.005% of a 1 mg nasal dose, is enough to reach the threshold of PRs inhibition. Accordingly, the administered dose may be as well low to minimise the exposure of extra-CNS districts.

In a preferred embodiment of the present invention mifepristone is administered at a daily nasal dose of 0.1-10 mg, preferably 0.5-5 mg, more preferably 0.75- 2.5 mg.

Once started, mifepristone nasal administration should continue in parallel with FSH administration until the targeted development of the follicles is reached, i.e. when a good number of follicles appear to be ready for the final maturation. In practice, the day of FSH withdrawal will also be the day of mifepristone withdrawal. Thereafter, the gonadotrophin surge will occur either spontaneously or following triggering with natural progesterone.

In a preferred embodiment of the present invention, once mifepristone is withdrawn, the gonadotrophin surge occurs spontaneously within 2 to 3 days.

In another preferred embodiment of the present invention, once mifepristone is withdrawn, the gonadorophin surge is stimulated by the administration of natural progesterone.

In a complementary aspect the present invention also provides a new pharmaceutical product containing an antiprogestin molecule, administered by the nasal route, for use in performing a COH cycle. Briefly, the cycle is started with the administration of the daily dose of FSH that is normally selected for that type of patient and is thereafter titrated according to the ovarian response as usually done. It is noted that no pituitary suppression regimen is established and that no LH supplementation is required as the pituitary will release LH as and when needed to balance the FSH activity. When the leading follicles reach the diameter of 16-18 mm, nasal mifepristone at very low doses is started in parallel with continuing FSH administration. Once the targeted development is reached, i.e. a good number of follicles are ready to ovulate, both FSH and mifepristone are discontinued. The administration of nasal progesterone starts on the first day of FSH/mifepristone withdrawal and continues for 48 hours while plasma LH is monitored to detect the initial rise of the surge. The oocytes pick-up is planned at 48 hours from the start of LH rise. Thereafter the oocytes are fertilised and the embryos transferred according to the usual practices. The embryo transfer does not need to be sustained with a luteal support treatment.

The new pharmaceutical product for use in COH cycle is innovative with respect to the state of the art in a series of aspects as follows:
1. Differently from the state of the art, the cycle is not started with a pituitary desensitisation treatment, neither it will include any GnRH antagonist later on. Besides saving resources, the avoidance of the pituitary suppression allows a stimulation cycle under pituitary control. Indeed, in these conditions the pituitary is expected to account for any changes of the endocrine milieu so to create the best conditions for the follicles growth.
2. As a consequence of 1, in the majority of patients there will be no need to include a LH supplementation in parallel to FSH administration. Indeed, the pituitary will be able to release LH pulses with the appropriate frequency, amplitude and LH isoform composition. Again, this is a saving in resources but also an increase of the quality of the stimulation because exogenous LH agonists administered under pituitary blockade, besides in part substituting the lacking hormone, also further interfere the feedbacks reaching the pituitary misleading also the minimal residual pituitary secretion.
3. Differently from the state of the art, the ovulatory gonadotropin surge will be natural, i.e. released by the pituitary, either spontaneously occurring after nasal mifepristone discontinuation or triggered by administration of natural progesterone.
4. Differently from the state of the art, in the majority of patients the embryo transfer will not require to be sustained with a luteal support. Indeed, the active pituitary regulation balancing the background endocrine milieu will also benefit the endometrium accounting for the disturbances generated by the supraphisiological FSH amounts and the supraphysiological estrogen levels.

As regards the use of natural progesterone for triggering the gonadotropin surge, it is to be noted that this will benefit the COH cycle in two different ways.

First, as largely explained herein, progesterone will activate the PR of the hypothalamus so far inhibited by mifepristone. Indeed, the inhibition is competitive, i.e. it is directly related to the relative concentration of progesterone and of its antagonist. Following discontinuation of mifepristone, its CNS concentration will start to decline while progesterone concentration rise under active dosing, which will cause a fast inversion of the receptors occupancy. The rapidity of such conversion will be directly proportional to the amount of progesterone delivered, i.e. to the amount and to the frequency of progesterone dosing. On the other side, it will be inversely correlated to the amount of mifepristone previously administered.

Second, in the physiologic cycle the LH surge acts by stimulating both granulosa cell progesterone production and granulosa exposure of PRs. In these conditions progesterone acts via genomic mechanisms involving nuclear progesterone receptors and non-genomic mechanisms by the activation of a putative membrane PR. A main effect of such follicular progesterone activation will be the loosening of the gap junctions of the oocyte cumulus that start the events leading to the release of the oocyte. Inasmuch, dosing progesterone to trigger the gonadatrophin surge will also start the events caused by the same surge and will be synergic with the effect of the surge on the final maturation and release of the oocyte.

As a consequence of the above arguments, the maximum dose of progesterone to be used to trigger the gonadotropin surge is not critical, i.e. it can be in excess on the minimal need, but it is critical to reach the minimal need at the CNS compartment. Moreover, aiming to well timed gonadotropin surge, it is important that threshold concentration of progesterone is reached at the hypothalamic level within a short time following FSH withdrawal with a sort of peak effect. This can be obtained by the intranasal delivery of progesterone, possibly at relatively high amounts by repeated doses at short intervals. On the other side, the excess of progesterone delivered as such and entering the bloodstream is not a problem, rather it benefits the effects of the gonadotropin surge occurring thereafter by somehow anticipating the effect on the cumulus cells that the LH peak is known to have.

In a preferred embodiment of the present invention the new pharmaceutical product for use in COH includes triggering of the gonadotrophins surge by delivery of progesterone to the CNS, preferably by the nasal route.

In another preferred embodiment of the present invention the new pharmaceutical product for use in COH includes administration of nasal progesterone starting from the first day of FSH and mifepristone withdrawal.

In another preferred embodiment of the present invention the new pharmaceutical product for use in COH includes administration of nasal progesterone at doses in excess to the minimal need to remove the mifepristone blockade and with repeated administrations in short distance such as 1-20 mg, preferably 2-10 mg, and at intervals of 8 hours, preferably 4 hours, more preferably 2 hours up to the confirmed occurrence of the gonadotrophin surge.

The occurrence of the gonadotrophin surge is confirmed by monitoring the plasma concentration of LH. The actual persistence of very high circulating LH in case of a natural cycle is in the range of 48 hours. Accordingly, aiming to sustain the surge over time, progesterone dosing will be changed as soon as there will be clear evidence of progressive rise of LH. Starting from this point progesterone will be administered at fixed 8 hours intervals up to completion of an overall progesterone treatment of 48 hours.

The follicular events following the occurrence of the surge take approximately 48 hours to complete, therefore the oocytes will be ready for the pick-up 48 hours later than the confirmed occurrence of the LH surge, i.e. within 72 hours from the first progesterone dose.

In alternative, the LH peak may be obtained without any further treatment, i.e. waiting for a longer time, likely 3 days, after FSH and mifepristone discontinuation and the oocyte pick-up is planned 48 hours following confirmed start of the surge. As a further alternative, a progesterone bolus and a hCG bolus are administered at time of FSH and mifepristone discontinuation and the oocyte pick-up is planned at 72 hours post-dosing.

The new pharmaceutical product described in the present invention, i.e. a progesterone antagonist such as mifepristone (RU486) formulated for intranasal (i.e. nose to brain) delivery, is easily suitable for manufacturing at industrial scale because: i) the active pharmaceutical ingredient is largely available in the market and not protected by active intellectual properties; ii) the formulation for intra-nasal administration can be achieved without recurring to difficult or expensive ingredients; iii) the technologies for manufacturing as a liquid formulation, scaling-up at industrial sizes and primary and secondary packaging suitable for nasal delivery are widely available in the state of the art; iv) the potential market demand, in the range of millions of daily doses per year, is compatible with the industrial economy scale.

Example 1, progesterone triggering: patients referring to a specialised human reproduction centre due to idiopathic infertility are administered FSH from day 2 of the follicular cycle at starting daily doses equal to 150 IU for patients with a Body Mass Index (BMI) < 25 and equal to 225 IU in patients with a BMI > 25 or carrying known resistance factors. No pituitary suppression is established neither a LH containing drug is associated to FSH. The cycle is monitored by transvaginal ultrasonographic scan (TV-US) starting from day 5 of stimulation and thereafter any other day and the dose of FSH is titrated according to the follicle growth. When at least 2 follicles reach the diameter of 16 mm mifepristone administration starts. Mifepristone is given at 1 mg daily doses by the nasal route while FSH administration continues at the doses suggested by the follicles response. At the first planned monitoring session in which the TV-US reveals at least 1 follicle having a diameter of 20 mm, FSH and mifepristone are discontinued and progesterone dosing starts on the same day. Progesterone is given by the nasal route at doses of 5 mg at 4 hours intervals for 20 hours (5 doses) and LH circulating level is checked at 24 hours (4 hours after the last progesterone dose): the LH peak is assumed as triggered for any value of circulating LH higher than 30 IU per litre. Patients whose LH is higher than such cut-off value undergo further administration of 5 mg intranasal progesterone at 8 hours intervals for another 24 hours. Patients below the LH cut-off value continue progesterone dosing at 4 hours intervals until the targeted LH cut-off value is reached, thereafter they complete the cycle with dosing at 8 hours intervals as previously described. The oocyte pick-up is planned at 24 hours after the last progesterone dose.

Example 2, no triggering: the FSH stimulation and the mifepristone nasal dosing as well as the monitoring procedure are performed as described in Example 1 and these drugs are discontinued according to the same criteria. At the first planned monitoring session in which the TV-US reveals at least 1 follicle having a diameter of 18 mm, mifepristone is discontinued whereas FSH dosing continues for another day and then is discontinued as well. Plasma LH levels are monitored at 12 hours interval starting from 24 hours following FSH discontinuation. The oocyte pick-up is planned at 48 hours following the first rise of plasma LH higher than 30 IU per litre.

Example 3, progesterone and hCG triggering: the FSH stimulation and the mifepristone nasal dosing as well as the monitoring procedure is performed as described in Example 1. Thereafter, at the first planned monitoring session in which the TV-US scan reveals at least 1 follicle having a diameter of 20 mm, FSH and mifepristone are discontinued and the LH surge is triggered with a bolus of 25 mg progesterone, either intranasal or injectable, and 5'000 IU of hCG. The oocyte pick-up is planned 72 hours later.

### References

1. Arslan, M et al. - Controlled ovarian hyperstimulation protocols for in vitro fertilization: two decades of experience after the birth of Elizabeth Carr. Fertil. Steril. 2005; 84(3): 555-69.
2. Hayden, C - GnRH analogues: applications in assisted reproductive techniques. Eur. J. Endocrinol. 2008; 159 (Suppl 1): S17-S25.
3. Humaiden, P et al. - GnRHa to trigger final oocyte maturation: a time to reconsider. Hum Reprod. 2009; 24(10): 2389-2394.
4. Micevych, P & Sinchak K - Estradiol regulation of progesterone synthesis in the brain. Mol. Cell Endocrinol. 2008; August 13; 290(1-2): 44-50.
5. Cadepond, F et al. - RU486 (mifepristone) : mechanisms of action and clinical uses. Annu. Reev. Med. 1997; 48: 129-156.
6. Permezel, JM et al. - Acute effects of progesterone and the antiprogestin on gonadotropin secretion in the follicular phase of the menstrual cycle. J. Clin. Endocrinol. Met. 1989; 68(5): 960-965.
7. Batista, MC et al. - Evidence for a critical role of progesterone in the regulation of the midcycle gonadotropin surge and ovulation. J. Clin. Endocrinol. Met. 1992; 74(3): 565-570.
8. Croxatto HB et al. - Follicle stimulating hormone-granulosa cell axis involvement in the antifolliculotrophic effect of low dose mifepristone (RU486). Human Reprod. 1995; 10(8): 1987-1991.
9. Coelingh Bennink, HJT & Bunschoten, EJ - EP1455831 - Method of controlled ovarian hyperstimulation and pharmaceutical kit for use in such method.
10.Andersen, AN et al. - Clinical outcome following stimulation with highly purified hMG or recombinant FSH in patients undergoing IVF: a randomized assessor-blind controlled trial. Hum. Reprod. 2006; 21(12): 3217-3227.
11. Pardridge, WM - The blood-brain barrier: the bottleneck in brain drug development. NeuroRx 2005; 2: 3-14.
12. Illum, L - Transport of drugs from the nasal cavity to the central nervous system. Eur. J. Pharm. Sci. 2000; 11: 1-18.
13. Thorne, RG - Delivery of insulin-like growth factor-1 to the rat brain and spinal cord along trigeminal pathway following intranasal administration. Neuroscience 2004; 127: 481-496.
14. Reger, MA et al. - Effect of intranasal insulin on cognition in memory impaired older adults: modulation by APOE genotype. Neurobiol. Aging 2006; 27: 451-458.
15.Yu, IP et al. - Intranasal recombinant human erythropoietin protects rats against focal cerebral ischemia. Neurosci. Letter 2005; 387: 5-10.
16. Jin, K et al. - Cerebral neurogenesis is induced by intranasal administration of growth factors. Annal. Neurol. 2003; 53: 405-409.
17. Ying, W et al. - Intranasal administration with NAD+ profoundly decreases brain injury in a rat model of transient focal ischemia. Front. Biosci. 2007; 12: 2728-2734.
18.Ying, W et al. - Therapeutic potential of NAD+ for treating neurological diseases. Future Neurol. 2007; 2: 129-132.
19. Romano Lagunas, GL et al. - Effectiveness of nasal vs oral estrogens in the climacteric syndrome (article in Spanish). Gynecol Obstet. Mex. 2007; 75(3): 127-32.
20. Cicinelli, E et al. - Administration of unmodified progesterone by nasal spray in fertile women. Gynecol. Endocrinol. 1995; 9(4): 289-293.
21. Escudero, EL et al. - Mifepristone is an effective oral alternative for the prevention of premature luteinizing hormone surges and/or premature luteinization in women undergoing controlled ovarian hyperstimulation for in vitro fertilization. J. Clin. Endocrinl. Metab. 2005; 90(4): 2081-2088.
22. Ulmann A. et al. - US 2010/261693 - A method for treating Cushing syndrome.
23. Hausknecht R. - US 2007/213306 - Methods, dosing regimens and medications using anti progestational agents for the treatment of disorders
24. Delabre, K et al. - In vivo evidence against the existence of antiprogestins disrupting receptor binding to DNA. Proc. Natl. Acad. Sci. 1993; 90: 4421-4425.

## Claims

1. A pharmaceutical product containing an antiprogestinic molecule, for use in the temporary suppression of the spontaneous midcycle gonadotrophin surge in women undergoing follicular stimulation for Assisted Reproduction (ART) purposes by delivery of said antiprogestinic molecule to the Central Nervous System (CNS), **characterized in that** it is to be administered by nasal route starting from the day the follicles reach the size of 16 mm in diameter.

2. The pharmaceutical product for use of claim 1 wherein the antiprogestinic molecule is mifepristone (RU486).

3. The pharmaceutical product for use according to any of the preceding claims, to be administered starting from the day the follicles reach the size of 18 mm in diameter.

4. The pharmaceutical product for use according to any of the preceding claims, to be administered not later then day 8 of stimulation, and to be withdrawn as soon as the targeted follicular development has been achieved and a spontaneous gonadotrophin surge is wanted.

5. The pharmaceutical product for use of claim 1, wherein the nasal daily dose is 0.1-10 mg mifepristone.

6. The pharmaceutical product for use of claim 1, wherein the nasal daily dose is 0.5-5 mg mifepristone.

7. The pharmaceutical product for use of claim 1, wherein the nasal daily dose is 0.75- 2.5 mg mifepristone.

8. The pharmaceutical product for use according to any of the preceding claims, wherein mifepristone is formulated as solution in organic solvents, including but not limited to, ethanol and dimethyl sulfoxide (DMSO) or as an organic oil solution together with other pharmaceutically acceptable ingredients.

9. The pharmaceutical product as defined in any of the preceding claims, for use in the timing modulation of the spontaneous gonadotrophin surge as part of follicular stimulation cycles for assisted reproduction purposes by delaying the occurrence of the gonadotrophin surge.

10. The pharmaceutical product for use of claim 9 wherein the pituitary gonadotrophin surge occurs spontaneously after mifepristone withdrawal without any triggering intervention.

11. The pharmaceutical product for use of claim 9 wherein the pituitary gonadotrophin surge is induced after mifepristone withdrawal by administration of natural progesterone by the nasal route.

12. The pharmaceutical product for use of claim 11 wherein natural progesterone is administered by the nasal route at single doses of 1-20 mg, preferably 2-10 mg.

13. The pharmaceutical product for use of claim 11 or 12 wherein natural progesterone is administered by the nasal route at intervals of 8 hours, preferably 4 hours, more preferably 2 hours and until the release of the LH surge starts as confirmed by the rise of LH plasma concentration.

14. The pharmaceutical product for use of claim 11, 12 or 13 wherein, following confirmed occurrence of the LH surge, progesterone is administered at 8 hour intervals up to completion of a total progesterone treatment of at least 48 hours.

15. The pharmaceutical product for use of claim 11, 12, 13 or 14 wherein the oocytes pick-up is performed 24 hours following intranasal progesterone discontinuation.

## Patentansprüche

1. Pharmazeutisches Produkt, das ein anti-progestines Molekül enthält, zur Verwendung bei der temporären Supprimierung des spontanen starken Anstiegs an Gonadotropin in der Zyklusmitte bei Frauen, die einer Follikel-Stimulierung für unterstützende Reproduktions-(ART) Zwecke durchlaufen, durch Lieferung des anti-progestinen Moleküls zu dem zentralen Nervensystem (ZNS), **dadurch gekennzeichnet, dass** es über die nasale Route beginnend mit dem Tag verabreicht wird, zu dem die Follikel eine Durchmessergröße von 16 mm erreichen.

2. Pharmazeutisches Produkt zur Verwendung nach Anspruch 1, worin das anti-progestine Molekül Mifepriston (RU486) ist.

3. Pharmazeutisches Produkt zur Verwendung nach einem der vorstehenden Ansprüche, zur Verabreichung beginnend an dem Tag, an dem die Follikel eine Durchmessergröße von 18 mm aufweisen.

4. Pharmazeutisches Produkt zur Verwendung nach einem der vorstehenden Ansprüche, zur Verabreichung nicht später als Tag 8 der Stimulierung, und dann Absetzen sobald die beabsichtigte Follikel-Entwicklung erreicht wurde und ein spontaner Anstieg an Gonadotropin erwünscht ist.

5. Pharmazeutisches Produkt zur Verwendung nach Anspruch 1, worin die tägliche nasale Dosis 0,1 - 10 mg Mifepriston ist.

6. Pharmazeutisches Produkt zur Verwendung nach Anspruch 1, worin die tägliche nasale Dosis 0,5 - 5 mg Mifepriston ist.

7. Pharmazeutisches Produkt zur Verwendung nach Anspruch 1, worin die tägliche nasale Dosis 0,75 - 2,5 mg Mifepriston ist.

8. Pharmazeutisches Produkt zur Verwendung nach einem der vorstehenden Ansprüche, worin Mifepriston als Lösung in organischen Lösungsmitteln formuliert wird, einschließlich, ohne darauf beschränkt zu sein, Ethanol und Dimethylsulfoxid (DMSO), oder als eine organische Öl-Lösung zusammen mit anderen pharmazeutisch annehmbaren Inhaltsstoffen.

9. Pharmazeutisches Produkt nach einem der vorstehenden Ansprüche zur Verwendung bei der zeitlichen Planung der Modulation des spontanen Anstiegs von Gonadotropin als Teil von Follikel-Stimulierungs-Zyklen für unterstützende Reproduktions-Zwecke durch Verzögern des Auftretens des Anstiegs an Gonadotropin.

10. Pharmazeutisches Produkt zur Verwendung nach Anspruch 9, worin der Anstieg des Hypophysen-Gonadotropins nach Absetzen von Mifepriston ohne auslösende Intervention spontan erfolgt.

11. Pharmazeutisches Produkt zur Verwendung nach Anspruch 9, worin der Anstieg des Hypophysen-Gonadotropins nach Absetzen von Mifepriston durch Verabreichen von natürlichem Progesteron über die nasale Route induziert wird.

12. Pharmazeutisches Produkt zur Verwendung nach Anspruch 11, worin natürliches Progesteron über die nasale Route in Einzeldosen von 1 - 20 mg, vorzugsweise 2 - 10 mg verabreicht wird.

13. Pharmazeutisches Produkt zur Verwendung nach Anspruch 11 oder 12, worin natürliches Progesteron über die nasale Route in Intervallen von 8 Stunden verabreicht wird, vorzugsweise 4 Stunden, mehr bevorzugt 2 Stunden und bis die Freisetzung des Anstiegs an LH beginnt, bestätigt durch den Anstieg der LH-Plasma-Konzentrationen.

14. Pharmazeutisches Produkt zur Verwendung nach Anspruch 11, 12 oder 13, worin nach bestätigtem erfolgtem Anstieg an LH, Progesteron in 8-Stunden Intervallen bis zur Beendigung der Gesamt-Progesteron-Behandlung von mindestens 48 Stunden verabreicht wird.

15. Pharmazeutisches Produkt zur Verwendung nach Anspruch 11, 12, 13 oder 14, worin die Oozyten-Aufnahme 24 Stunden nach dem Absetzen des intranasalen Progesterons durchgeführt wird.

## Revendications

1. Produit pharmaceutique contenant une molécule antiprogestative, pour une utilisation dans la suppression temporaire du pic spontané de gonadotrophine en milieu de cycle chez les femmes subissant une stimulation folliculaire à des fins de procréation assistée (ART) par administration de ladite molécule antiprogestative au système nerveux central (SNC), **caractérisé en ce qu'**il doit être administré par voie nasale en commençant le jour où les follicules atteignent la taille de 16 mm de diamètre.

2. Produit pharmaceutique pour une utilisation selon la revendication 1, dans lequel la molécule antiprogestative est la mifépristone (RU486).

3. Produit pharmaceutique pour une utilisation selon l'une quelconque des revendications précédentes, à administrer en commençant le jour où les follicules atteignent la taille de 18 mm de diamètre.

4. Produit pharmaceutique pour une utilisation selon l'une quelconque des revendications précédentes, à administrer pas plus tard qu'au jour 8 de la stimulation, et à arrêter dès que le développement folliculaire ciblé a été atteint et qu'un pic spontané de gonadotrophine est voulu.

5. Produit pharmaceutique pour une utilisation selon la revendication 1, dans lequel la dose quotidienne nasale est de 0,1 à 10 mg de mifépristone.

6. Produit pharmaceutique pour une utilisation selon la revendication 1, dans lequel la dose quotidienne nasale est de 0,5 à 5 mg de mifépristone.

7. Produit pharmaceutique pour une utilisation selon la revendication 1, dans lequel la dose quotidienne nasale est de 0,75 à 2,5 mg de mifépristone.

8. Produit pharmaceutique pour une utilisation selon l'une quelconque des revendications précédentes, dans lequel la mifépristone est formulée sous forme de solution dans des solvants organiques, comprenant, mais sans limitation, l'éthanol et le diméthylsulfoxyde (DMSO) ou sous forme de solution huileuse organique avec d'autres ingrédients pharmaceutiquement acceptables.

9. Produit pharmaceutique tel que défini selon l'une quelconque des revendications précédentes, pour une utilisation dans la modulation du timing du pic spontané de gonadotrophine en tant que partie des cycles de stimulation folliculaire à des fins de procréation assistée en retardant la survenue du pic de gonadotrophine.

10. Produit pharmaceutique pour une utilisation selon la revendication 9, dans lequel le pic de gonadotrophine pituitaire se produit spontanément après l'arrêt de la mifépristone sans aucune intervention de déclenchement.

11. Produit pharmaceutique pour une utilisation selon la revendication 9, dans lequel le pic de gonadotrophine pituitaire est induit après l'arrêt de la mifépristone par l'administration de progestérone naturelle par voie nasale.

12. Produit pharmaceutique pour une utilisation selon la revendication 11, dans lequel de la progestérone naturelle est administrée par voie nasale à des doses unitaires de 1 à 20 mg, de préférence de 2 à 10 mg.

13. Produit pharmaceutique pour une utilisation selon la revendication 11 ou 12, dans lequel la progestérone naturelle est administrée par voie nasale à des intervalles de 8 heures, de préférence de 4 heures, plus préférablement de 2 heures et jusqu'à l'initiation de la libération du pic de LH confirmée par l'augmentation de la concentration plasmatique de LH.

14. Produit pharmaceutique pour une utilisation selon la revendication 11, 12 ou 13, dans lequel après confirmation de la survenue du pic de LH, de la progestérone est administrée à des intervalles de 8 heures jusqu'à l'achèvement du traitement de progestérone total d'au moins 48 heures.

15. Produit pharmaceutique pour une utilisation selon la revendication 11, 12, 13 ou 14, dans lequel le prélèvement des oocytes est réalisé 24 heures après l'arrêt de l'administration de progestérone par voie intranasale.
